# EUROPÄISCHE PATENTSCHRIFT

(11) **EP 0 898 965 B1**
(45) Veröffentlichungstag und Bekanntmachung des Hinweises auf die Patenterteilung: **29.03.2006**
(21) Anmeldenummer: 98110302.1
(22) Anmeldetag: 05.06.1998
(51) Int. Cl.: A61K 31/505

(54) **Verwendung von 5-methyl-(6S)-tetrahydrofolsäure zur Vorbeugung von Neuralrohrdefekten**
Use of 5-methyl-(6S)-tetrahydrofolic acid for preventing neural tube defects
Utilisation de l'acid 5-methyl-(6S)-tétrahydrofolique pour la prevention des defauts du tube neural

(30) Priorität: 13.06.1997 CH 145697
(43) Veröffentlichungstag der Anmeldung: 03.03.1999
(73) Patentinhaber: Merck Eprova AG, 8200 Schaffhausen (CH)
(72) Erfinder: Müller, Hans Rudolf, 8207 Schaffhausen (CH); Moser, Rudolf, 8200 Schaffhausen (CH); Ulmann, Martin, 8447 Dachsen (CH)

(56) Entgegenhaltungen:
- EP-A- 0 595 005
- EP-A- 0 773 221
- WO-A-91/17660
- WO-A-97/27764
- WO-A-98/19690
- GB-A- 1 350 160
- US-A- 5 059 595
- US-A- 5 300 505
- US-A- 5 455 236
- LUCOCK, M. D. ET AL: "The methylfolate axis neural tube defects: In vitro characterization and clinical investigation" BIOCHEMICAL MEDICINE AND METABOLIC BIOLOGY, Bd. 52, 1994, Seiten 101-114, XP000916705
- MEROPOL, N. J. ET AL: "A Phase II and pharmacokinetic study of 6S-Leucovirin plus 5 fluorouracil in patients with colorectal carcinoma" INVEST. NEW DRUGS, Bd. 13, Nr. 2, 1995, Seiten 149-155, XP000916689
- DUELL P B ET AL: "HOMOCYST(E)INE: AN IMPORTANT RISK FACTOR FOR ATHEROSCLEROTIC VASCULAR DISEASE" CURRENT OPINION IN LIPIDOLOGY,GB,LONDON, Bd. 8, Nr. 1, Februar 1997 (1997-02), Seiten 28-34, XP000853544 ISSN: 0957-9672

## Beschreibung

Diese Erfindung bezieht sich auf die Verwendung von 5-Methyl-(6S)-tetrahydrofolsäure oder pharmazeutisch verträglichen Salzen von 5-Methyl-(6S)-tetrahydrofolsäure zur Herstellung einer pharmazeutischen Zubereitung geeignet zur Vorbeugung von Neuralrohr-Defekten.

Als Arzneimittel wurden Tetrahydrofolate bisher vorwiegend als Calcium-Salz der 5-Formyl-5,6,7,8-tetrahydrofolsäure (Leucovorin) oder der 5-Methyl-5,6,7,8-tetrahydrofolsäure verwendet zur Behandlung von megaloblastischer Folsäure-Anämie, als Antidot zur Verstärkung der Verträglichkeit von Folsäure-Antagonisten, speziell von Aminopterin und Methotrexat in der Krebstherapie ("Antifolate rescue"), zur Verstärkung des therapeutischen Effektes von fluorierten Pyrimidinen und der Behandlung von Autoimmunkrankheiten wie Psoriasis und rheumatischer Arthritis, zur Verstärkung der Verträglichkeit von bestimmten Antiparasitika, etwa Trimethoprim-Sulfamethoxazol sowie zur Verminderung der Toxizität von Dideazatetrahydrofolaten in der Chemotherapie.

Homocystein ist eine Thiol-enthaltende Aminosäure, die bei der Demethylierung von Methionin entsteht. In Körperflüssigkeiten liegt Homocystein in oxidierter Form als Disulfid (Homocystin), gemischte Disulfide und als cyclisiertes Oxidationsprodukt (Homocystein-Thiolacton) vor.

Hyperhomocysteinämie ist eine klinische Störung, die verschiedene angeborene oder erworbene Ursachen haben kann. Diese Störungen haben eine erhöhte Konzentration von Homocystein im Blut und im Ham zur Folge.
Die häufigste Form von Hyperhomocysteinämie resultiert aus einem Defekt der Cystathion-β-Synthase, einem Enzym im B₆-abhängigen Transulfurierungspfad, in dem Homocystein über Cystathionin in Cystein umgewandelt wird. Eine andere Form basiert auf einem Defekt der 5,10-Methylentetrahydrofolat-Reduktase, die das Substrat, 5-Methyl-(6S)-tetrahydrofolsäure, für die B₁₂-abhängige Umwandlung von Homocystein zu Methionin liefert. Hyperhomocysteinämie kann auch als Folge von Funktionsstörungen der Niere auftreten. In all diesen Fällen bedeutet der Begriff Hyperhomocysteinämie eine vorübergehende oder permanente Erhöhung des Homocystein-Spiegels im Blut, der z. T. begleitet wird von einer erhöhten Homocystein-Ausscheidung im Ham.
Hyperhomocysteinämie hat eine Reihe von Krankheiten zur Folge, die sich in schweren vaskularen, okularen, neurologischen und skeletalen Störungen zeigen.
Verschiedene klinische Studien zeigen einen klaren Zusammenhang zwischen einem erhöhten Homocystein-Level im Serum und der Entwicklung von Herz-/Kreislauferkrankungen. Homocysteinämie wird als unabhängiger Risikofaktor bei Herz-/Kreislauferkrankungen angesehen. Allgemeine Angaben können gefunden werden in K.L. Resch ed, Risikofaktor Homocystein Daten-Fakten-Strategien, Gesellschaft für Medizinische Information ISBN 3-980 45 36-0-X. Im Zusammenhang zwischen Hyperhomocysteinämie und Arteriosklerose sei auf L.J. Fortin et al, Clinical Biochemistry, Vol. 28(2), 1995, Seiten 155-162 hingewiesen. Hyperhomocysteinämie und Neuralrohr-Defekte wurden beschrieben von J.L. Mills et al, Supplement Publication to the Ceres-Forum on June 14, 1995, 1996, Seiten 756S-760S.

Die Verwendung von 5-Methyl-(6S)-tetrahydrofolsäure oder pharmazeutisch verträglichen Salzen von 5-Methyl-(6S)-tetrahydrofolsäure zur Herstellung einer pharmazeutischen Zubereitung geeignet zur Vorbeugung von Neuralrohr-Defekten wurde bisher weder vorgeschlagen noch beschrieben.

Es wurde gefunden, dass die Verwendung von pharmazeutischen Zubereitungen enthaltend 5-Methyl-(6S)-tetrahydrofolsäure oder pharmazeutisch verträgliche Salze von 5-Methyl-(6S)-tetrahydrofolsäure zur Vorbeugung von Neuralrohr-Defekten geeignet ist.

5-Methyl-(6S)-tetrahydrofolsäure ist direkt als Methyl-Donor an der Übertragung der Methylgruppe von Homocystein zu Methionin beteiligt. Speziell gilt dies bei vorhandener Methylentetrahydrofolat-Reduktase-Defizienz, wobei Defizienz Störungen wie z. B. eingeschränkte Funktionalität oder Mangel an Aktivität bedeutet

Als häufigstes Beispiel einer Methylentetrahydrofolat-Reduktase-Defizienz sei hier das Vorhandensein thermolabiler Methylentetrahydrofolat-Reduktase erwähnt. Unter diesen Umständen können andere Tetrahydrofolate nur beschränkt umgewandelt und damit in der Methylierungsreaktion mit eingesetzt werden.

Pharmazeutisch verträgliche Salze sollten sowohl pharmakologisch wie auch pharmazeutisch verträglich sein. Solche pharmakologisch und pharmazeutisch verträglicile Salze können Alkali- oder Erdalkalimetall-Salze sein, vorzugsweise Natrium-, Kalium-, Magnesium oder Calcium-Salze.

Pharmazeutische Zubereitungen beziehen sich auf enterale (z. B. oral, sublingual oder rektal), parenterale oder topische (z. B. transdermale) Formen. Als Träger können organische oder anorganische Substanzen verwendet werden, die nicht mit der aktiven Wirksubstanz reagieren, z. B. Wasser, Oel, Benzylalkohol, Polyethylenglycol, Glycerintriacetat oder andere Fettsäureglyceride, Gelatine, Lecithin, Cyclodextrine, Kohlenhydrate wie Lactobiose oder Stärke, Magnesiumstearat, Talk oder Cellulose. Bevorzugt bei der oralen Anwendung sind Tabletten, Dragees, Kapseln, Pulver, Sirup, Konzentrate oder Tropfen, für die rektale Anwendung bevorzugt sind Zäpfchen, zur parenteralen Anwendung bevorzugt eingesetzt werden Lösungen, wasser- oder oelbasierend oder Lyophilisate.
Ebenfalls anwendbar sind Suspensionen, Emulsionen oder Implantate und für die topische Anwendung Pflaster oder Cremes.
Pharmazeutische Zubereitungen für die parenterale Anwendung beinhalten sterile wässrige und nichtwässrige Injektionslösungen der pharmazeutisch aktiven Verbindungen, die vorzugsweise isotonisch zum Blut des Empfängers sind.

Diese Zubereitungen können Stabilisatoren, Additive für die kontrollierte Freisetzung der pharmazeutisch aktiven Verbindungen, Anüoxidantien, Puffer, Bakteriostatikas und Hilfsstoffe zur Einstellung einer isotonischen Lösung beinhalten. Wässrige und nichtwässrige sterile Suspensionen können Suspensionszusatzstoffe und Verdickungsmittel beinhalten. Die pharmazeutische Zubereitung kann als Einfachdosis- oder als Mehrfachdosis-Behälter vorhanden sein, zum Beispiel als verschweisste Ampullen und kann als gefriergetrocknetes (lyophilisiertes) Produkt gelagert und bei Bedarf mit steriler Flüssigkeit, zum Beispiel Wasser oder Salzlösung für den Gebrauch vorbereitet werden. In derselben Art und Weise können auch steriles Pulver, Granulat oder Tabletten verwendet werden. Alle pharmazeutischen Zubereitungen können zusätzlich separat oder synergistisch wirkende aktive Verbindungen enthalten. Zu erwähnen sind hier Vitamine, speziell aus der in dieser Anwendung synergistisch wirkenden Vitamin B-Gruppe, wie B₆ und/oder B₁₂. Dabei kann Vitamin B₆ in einer Dosis zwischen 1 mg und 20 mg, vorzugsweise bei normal dosierter Anwendung zwischen 1 mg und 6 mg pro Tag und bei hoch dosierter Anwendung zwischen 6 mg und 20 mg pro Tag eingesetzt werden. Vitamin B₁₂ kann in einer Dosis zwischen 0.001 mg und 0.5 mg, vorzugsweise bei normal dosierter Anwendung zwischen 0.001 mg und 0.15 mg pro Tag und bei hoch dosierter Anwendung zwischen 0.15 und 0.5 mg pro Tag eingesetzt werden.

Die pharmazeutische Zubereitung beinhaltet zwischen 0.001 mg und 1'000 mg der aktiven Wirksubstanz pro Dosis. In der Prophylaxe werden Zubereitungen enthaltend vorzugsweise zwischen 5 µg und 1'000 µg der aktiven Wirksubstanz pro Dosis eingesetzt. In der Therapie werden Zubereitungen enthaltend vorzugsweise zwischen 0.1 mg und 200 mg der aktiven Wirksubstanz pro Dosis eingesetzt.
Die Dosierung hängt ab von der Therapieform, von der Anwendungsform der pharmazeutischen Zubereitung, vom Alter, Gewicht, der Ernährung und Zustand des Patienten. Die Behandlung kann mit tiefer Dosierung unterhalb der optimalen Menge begonnen und bis zur Erreichung des optimalen Effektes gesteigert werden. Bevorzugt kann sich die Dosierungen in der Prophylaxe zwischen 5 µg und 1'000 µg pro Tag, im speziellen zwischen 50 µg und 500 µg pro Tag bewegen. Optimale Dosierungen in der Therapie bewegen sich zwischen 0.1 mg und 100 mg pro Tag, im speziellen zwischen 0.5 mg und 5 mg pro Tag. Die Anwendung kann entweder als Einmal-Gabe oder als wiederholte Dosierung erfolgen.

### Beispiele zur Illustrierung der Erfindung

### Beispiel 1

### Zäpfchen enthaltend 60 mg 5-Methyl-(6S)-tetrahydrofolsäure

Eine Mischung von 632 g 5-Methyl-(6S)-tetrahydrofolsäure-Calciumsalz-Pentahydrat (entsprechend 500 g 5-Methyl-(6S)-tetrahydrofolsäure), 50 g Hydroxypropylcellulose und 2 kg semisynthetische Glyceride werden zu Suppositorien geschmolzen, so dass jedes Zäpfchen 500 mg 5-Methyl-(6S)-tetrahydrofolsäure enthält.

### Beispiel 2

### Injektionslösung enthaltend 0.5 mg 5-Methyl-(6S)-tetrahydrofolsäure

0.5 g 5-Methyl-(6S)-tetrahydrofolsäure, 10 g Glutathion, 30 g Zitronensäure, 160 g Mannitol, 1 g Methyl-p-hydroxybenzoesäure, 17.7 g Natriumhydroxid (oder die notwendige Menge um einen pH-Wert der Lösung von 7.3 bis 7.8 einzustellen) werden auf 3 Liter Wasser zur Injektion gelöst und in Ampullen abgefüllt, so dass jede Ampulle 0.5 mg 5-Methyl-(6S)-tetrahydrofolsäure enthält.

### Beispiel 3

### Injizierbares Lyophilisat enthaltend 10 µg 5-Methyl-(6S)-tetrahydrofolsäure

Eine Lösung von 10 mg 5-Methyl-(6S)-tetrahydrafalsäure-Natriumsatz in 1'000 ml doppelt destilliertem Wasser wird unter Schutzgas in Ampullen stertlfiltriert und lyophilisiert, so dass jede Ampulle 10 µg 5-Methyl-(6S)-tetrahydrofolsäure enthält.

Tetrahydrofolsäure ist sehr sauerstoffempfindlich, daher muss strikte sauerstoff-frei gearbeitet werden. Der Einsatz eines Oxidationsschutzmittels wie Ascorbinsäure kann notwendig sein.

### Beispiel 4

### Tablette enthaltend 15 mg 5-Methyl-(6S)-tetrahydrofolsäure

Eine Mischung von 19.18 g 5-Methyl-(6S)-tetrahydrofolsäure-Caiciumsalz-Pentahydrat (entsprechend 15 g 5-Methyl-(6S)-tetrahydrofolsäure), 120 g Lactose, 21.5 g Maisstärke. 7.08 g Acetylcellulose, 2.28 g Diethylphthalat, 0.64 g Silicone HK-15 und 2 g Magnesiumstearat werden zu Tabletten gepresst, so dass jede Tablette 15 mg 5-Methyl-(6S)-tetrahydrofolsäure enthält.

Die Tablette kann beschichtet als Filmtablette oder gemahlen und in Kapseln abgefüllt verwendet werden.

### Beispiel 5

### Tabletten enthaltend 15 mg 5-Methyl-(6S)-tetrahydrofolsäure

In analoger Weise, wie im Beispiel 8 beschrieben, werden Tabletten enthaltend 15 mg 5-Methyl-(6S)-tetrahydrofolsäure mit Maisstärke, Milchzucker, Magnesiumstearat, Polyethylenglycol 6000, Polymetacrylat, Polysorbit 80, Dimethylpolysiloxan. Natriumhydroxid und Talk hergestellt.

### Beisplel 6

### Kombinationspräparat aus 5-Methyl-(6S)-tetrahydrofolsäure, Vitamin B₆ und Vitamin B₁₂

Für Präparate zur oralen Anwendung wird eine Filmtablette formuliert, die folgende Bestandteile enthält:

| | |
|---|---|
| 0.4 mg | 5-Methyl-(6S)-tetrahydrofolsäure |
| 3 mg | Vitamin B₆ |
| 0.002 mg | Vitamin B₁₂, pharmazeutisch verträgliche Hilfsstoffe |

Das Kombinationspräparat kann auch als Lösung z. B. für die parenterale Anwendung formuliert werden.

## Patentansprüche

1. Verwendung von 5-Methyl-(6S)-tetrahydrofolsäure oder pharmazeutisch verträglichen Salzen von 5-Methyl-(6S)-tetrahydrofolsäure zur Herstellung einer pharmazeutischen Zubereitung zur Vorbeugung von Neuralrohr-Defekten.

2. Verwendung von 5-Methyl-(6S)-tetrahydrofolsäure oder phannazeutisch verträglichen Salzen von 5-Methyl-(6S)-tetrahydrofolsäure zur Herstellung einer pharmazeutischen Zubereitung gemäss Anspruch 1, **dadurch gekennzeichnet, dass** die Anwendung bei vorhandener thermolabiler Methylentetrahydofolat-Reductase erfolgt.

## Claims

1. Use of 5-methyl-(6S)-tetrahydrofolic acid or pharmaceutically compatible salts of 5-methyl-(6S)-tetrahydrofolic acid for the production of a pharmaceutical preparation suitable for the prevention of medullary tube defects.

2. Use of 5-methyl-(6S)-tetrahydrofolic acid or pharmaceutically compatible salts of 5-methyl-(6S)-tetrahydrofolic acid for the production of a pharmaceutical preparation according to Claim 1, **characterized in that** the application is made in the presence of thermolabile methylenetetrahydrofolate reductase.

## Revendications

1. Utilisation d'acide 5-méthyl-(6S)-tétrahydrofolique ou de sels pharmaceutiquement compatibles de l'acide 5-méthyl-(6S)-tétrahydrofolique pour la fabrication d'une préparation pharmaceutique convenant à la prévention de défauts du tube neural.

2. Utilisation d'acide 5-méthyl-(6S)-tétrahydrofolique ou de sels pharmaceutiquement compatibles de l'acide 5-méthyl-(6S)-tétrahydrofolique pour la fabrication d'une préparation pharmaceutique selon la revendication 1, **caractérisée en ce que** l'utilisation a lieu en présence de méthylènehydrofolate-réductase thermolabile.
